# FASCICULE DE BREVET EUROPEEN

(11) **EP 2 043 532 B1**
(45) Date de publication et mention de la délivrance du brevet: **26.12.2018**
(21) Numéro de dépôt: 07805016.8
(22) Date de dépôt: 26.06.2007
(51) Int. Cl.: A61B 17/43, A61B 17/435

(54) **SYSTEME INTRA-UTERIN RECUPERABLE**
ENTNEHMBARES INTRAUTERINES SYSTEM
RECOVERABLE INTRA-UTERINE SYSTEM

(30) Priorité: 21.07.2006 FR 0653069
(43) Date de publication de la demande: 08.04.2009
(73) Titulaire: Anecova SA, 1015 Lausanne (CH)
(72) Inventeur: MOCK, Pascal, CH-1205 Genève (CH); BOUCHE, Nicolas, CH-1619 Les Paccots (CH); LE GOFF, Philippe, CH-1066 Epalinges (CH)
(74) Mandataire: Santarelli
(86) Numéro de dépôt international: PCT/IB2007/002913
(87) Numéro de publication internationale: WO 2008/012685

(56) Documents cités:
- EP-A- 1 639 972
- DE-A1- 3 438 349
- US-A- 3 656 483
- US-A- 4 312 347

## Description

La présente invention concerne un système intra-utérin récupérable.

De manière générale, elle concerne la stabilisation et le maintien en position d'un système intra-utérin récupérable, utilisé dans les processus de procréation médicalement assistée.

Les systèmes intra-utérins implantables et récupérables par les voies naturelles sont connus depuis plusieurs années.

De manière générale, ces systèmes peuvent être classés en trois grandes familles selon leur indication ou leur mode d'action. Parmi celles-ci, les dispositifs ayant une indication anti-conceptuelle, tels que des stérilets, représentent la grande majorité des cas et constituent la première catégorie.

La seconde catégorie est constituée des capsules chargées d'une substance thérapeutique et placées dans la cavité utérine pour diffusion et action dans l'organisme.

Enfin, la troisième catégorie est constituée de dispositifs intra-utérins indiqués pour l'assistance in vivo dans les processus de procréation médicalement assistée. On connaît ainsi un dispositif intra-utérin tel que décrit notamment dans le document WO 03/011200, destiné à être placé dans la cavité utérine pour une période comprise entre quelques heures et quelques jours, et permettant un développement préimplantatoire d'un embryon in vivo.

Pour chacune de ces catégories de dispositifs, il est primordial que l'objet placé par les voies naturelles dans la cavité utérine puisse rester positionné à l'endroit déterminé pendant la durée de l'indication et ensuite récupéré par les mêmes voies naturelles.

Pour répondre à ces spécifications, les dispositifs concernés doivent répondre à une double contrainte, a priori contradictoire. D'une part ces dispositifs doivent être à la fois fins et peu encombrants pour être placés dans la cavité utérine puis extraits, par les voies naturelles, ce qui impose de franchir le canal isthmique du col utérin. D'autre part, ils doivent être suffisamment encombrants pour se stabiliser dans la cavité utérine en évitant d'être expulsés par les contractions naturelles de l'utérus.

De nombreuses variantes et formes ont été proposées dans l'état de la technique pour répondre à cette double contrainte, mais exclusivement orientées pour des applications appartenant aux première et deuxième catégories de dispositifs présentées ci-dessus.

Ainsi, on connait dans le document US 3 656 483 un système intra-utérin adapté à contenir un médicament, avec des bras élastiques adaptés à être libérés après introduction du système dans l'utérus au moyen d'un cathéter.

Or, pour la troisième catégorie de dispositifs utilisés dans les processus de procréation in vivo médicalement assistée, une contrainte additionnelle apparaît dès lors qu'il est primordial de préserver l'intégrité de la paroi endométriale. En effet, une implantation embryonnaire doit avoir lieu durant le même cycle menstruel de la patiente et l'endomètre ne doit pas être altéré d'une quelconque manière avant l'implantation de l'embryon.

En particulier les systèmes de stabilisation existant dans le domaine de la contraception, et notamment pour le maintien en position d'un stérilet dans l'utérus, ne sont pas satisfaisants. En effet, ils peuvent endommager l'endomètre, non seulement pendant le maintien en position du dispositif dans la cavité utérine, en exerçant une pression constante contre l'endomètre (provoquant des phénomènes de contractions, avec éventuellement activation hormonale), mais aussi lors du retrait du dispositif, les éléments de maintien venant frotter sur une grande longueur de la paroi endométriale, provoquant ainsi des microlésions ou des inductions inflammatoires, ou encore des saignements.

Par ailleurs, le document WO 03/011200 décrit un dispositif intra-utérin portant des ailettes de stabilisation à une extrémité distale du logement. Ces ailettes de stabilisation sont susceptibles d'endommager la paroi endométriale, notamment lors du retrait du dispositif et de diminuer de ce fait les chances de réussite de l'implantation subséquente de l'embryon.

La présente invention a pour but de proposer un système intra-utérin récupérable permettant de résoudre les inconvénients précités et notamment de garantir le maintien en position dans la cavité utérine du système tout en préservant l'intégrité de l'endomètre, notamment lors du retrait du système.

À cet effet, la présente invention concerne un système intra-utérin récupérable comprenant un logement adapté à contenir un ou plusieurs éléments choisis parmi le groupe comprenant un embryon, des gamètes mâles et/ou femelles, un ovocyte fécondé, un ovule non fécondé et une combinaison de ces éléments, le logement présentant suivant un axe, une extrémité distale et une extrémité proximale, et comprenant un dispositif de maintien du système intra-utérin récupérable dans l'utérus tel que défini dans la revendication 1.

Le dispositif de maintien est agencé à l'extrémité proximale du logement et comporte au moins un bras de retenue dans la cavité utérine adapté à prendre au moins deux positions :
- une position libre de maintien dans laquelle ledit au moins un bras de retenue est écarté dudit axe ; et
- une position de retrait dans laquelle ledit au moins un bras de retenue est sensiblement parallèle audit axe.

Ainsi, grâce à l'agencement du dispositif de maintien à l'extrémité proximale du logement, le maintien en position du système introduit dans l'utérus est réalisé au niveau du canal isthmique et la surface de contact entre le ou les bras de retenue du dispositif de maintien avec la paroi endométriale est réduite. Ces caractéristiques ont pour effets bénéfiques de diminuer la pression induite par le système intra-utérin sur la cavité utérine.

Par ailleurs, lors du retrait du dispositif, le bras de retenue s'étendant sensiblement dans l'axe du logement, les frottements sur la paroi de l'endomètre au-delà du canal isthmique sont très limités, voire inexistants.

En outre, grâce à l'agencement du dispositif de maintien dans la cavité utérine à l'extrémité proximale du logement, la partie active du système, constituée par le logement, est positionnée au-dessus du ou des bras de retenue dans la cavité utérine, et ainsi intégralement incluse dans la cavité utérine.

Par ailleurs, lorsque le système est placé dans la cavité utérine avec son dispositif de maintien associé, le ou les bras de retenue s'étendent dans la cavité utérine, au-delà du col de l'utérus, et ne sont pas susceptibles d'obstruer le col de l'utérus et de gêner l'écoulement du fluide existant entre l'utérus et le vagin.

Selon une caractéristique particulière de l'invention, ledit au moins un bras de retenue est adapté à prendre en outre une position d'introduction dans laquelle ledit au moins bras de retenue est sensiblement parallèle audit axe et dans le prolongement dudit logement.

Grâce à cet agencement particulier du dispositif de maintien, le bras de retenue s'étendant dans le prolongement du logement, l'ensemble du système peut être introduit dans un cathéter de transfert traditionnel utilisé pour introduire le système intra-utérin récupérable à l'intérieur de la cavité utérine au-delà du col de l'utérus.

Dès lors que le bras s'étend dans le prolongement du logement, le dispositif de maintien ne forme pas de surépaisseur autour du logement et peut être introduit dans un cathéter de transfert de faible diamètre interne, et par là même, de faible diamètre externe, adapté au passage du col de l'utérus.

Selon une caractéristique avantageuse de l'invention, ledit au moins un bras de retenue est agencé sur des moyens élastiques ayant une force de rappel adaptée à maintenir ledit au moins un bras de retenue dans la position libre de maintien.

Grâce à la présence de ces moyens élastiques, le ou les bras de dispositif de maintien sont automatiquement placés dans leur position libre de maintien lorsque le système intra-utérin récupérable est introduit dans la cavité utérine, après retrait du cathéter de transfert utilisé pour son introduction.

Le maintien en position du système intra-utérin récupérable est ainsi réalisé de manière fiable et naturelle sans requérir de manipulation additionnelle de la part du praticien.

Selon un autre aspect, l'invention vise également un ensemble cathéter de transfert et système intra-utérin dans lequel le système est logé dans le cathéter.

Ainsi, le logement associé au dispositif de maintien peuvent être placés dans l'utérus grâce au cathéter de transfert.

D'autres particularités et avantages de l'invention apparaîtront encore dans la description ci-après.

Aux dessins annexés, donnés à titre d'exemples non limitatifs :
- la figure 1 est une vue schématique d'un système intra-utérin récupérable conforme à un mode de réalisation de l'invention ;
- la figure 2 est un vue en perspective illustrant schématiquement le montage d'un système intra-utérin récupérable de la figure 1 dans un cathéter de transfert ;
- la figure 3 est une vue en coupe illustrant schématiquement le système intra-utérin récupérable de la figure 1 lors de son introduction dans la cavité utérine ;
- la figure 4 est une figure analogue à la figure 3 illustrant le système intra-utérin récupérable dans la cavité utérine ; et
- la figure 5 est une vue analogue aux figures 3 et 4 illustrant le système intra-utérin récupérable lors de son retrait de la cavité utérine.

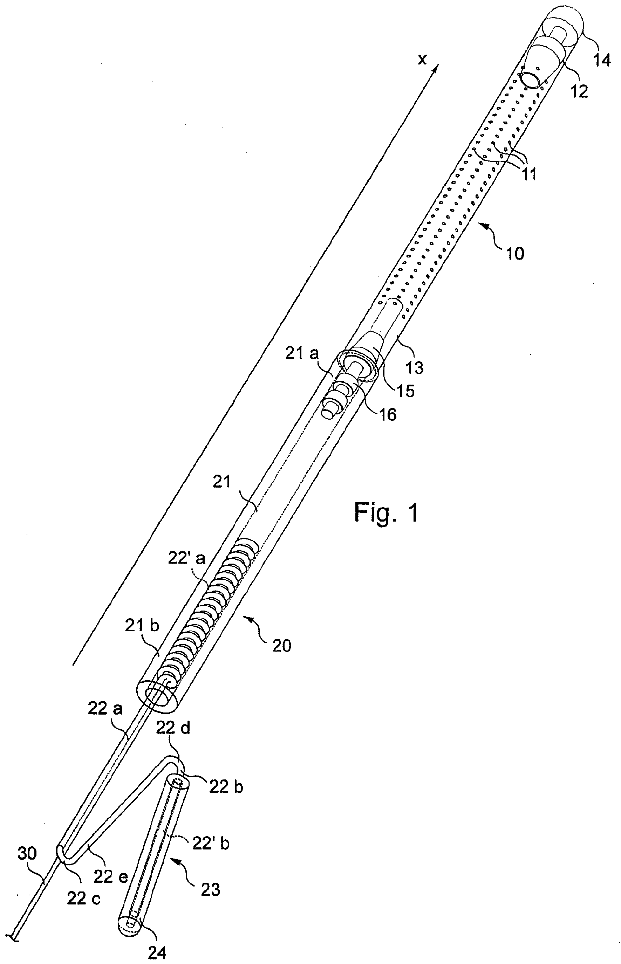

Comme illustré à la figure 1, le système intra-utérin récupérable comprend tout d'abord un logement 10 destiné à être placé dans l'utérus.

Ce logement 10 est destiné à être placé dans la cavité utérine pour une période comprise entre quelques heures et quelques jours en vue d'un développement préimplantatoire d'un embryon in vivo.

A cet effet, le logement 10 est adapté à contenir un embryon, des gamètes mâles et/ou femelles, un ovocyte fécondé, un ovule non fécondé ou encore une combinaison de ces différents éléments.

Ce logement 10 peut être réalisé en silicone et être pourvu dans sa paroi d'une série de perforations 11 permettant une interaction entre l'intérieur du logement et le milieu extérieur dans lequel est placé ce logement 10, c'est-à-dire le milieu utérin.

Comme bien illustré à la figure 1, ce logement 10 est de forme allongée suivant un axe X.

Dans ce mode de réalisation, le logement 10 est constitué d'un tube cylindrique allongé.

A titre d'exemples non limitatifs, la longueur du logement selon la direction X peut être égale sensiblement à 10 mm, le diamètre intérieur du logement 10 étant compris entre 0,4 et 0,5 mm et le diamètre extérieur du logement cylindrique 10 étant compris entre 0,7 et 0,8 mm.

Ce logement allongé 10 présente ainsi, suivant son sens d'introduction selon l'axe X dans une cavité utérine, une extrémité distale 12 et une extrémité proximale 13.

Le logement 10 comporte à son extrémité distale 12 un bouchon 14, par exemple en titane, fixé par ajustement à l'extrémité du logement 10 et permettant d'obturer celle-ci. Ce bouchon 14 peut être retiré après utilisation du système intra-utérin afin d'extraire les embryons et/ou éléments qui ont été placés temporairement dans la cavité utérine, en vue notamment de leur sélection et réimplantation dans la cavité utérine.

Le logement 10 comporte également un bouchon proximal 15 à son extrémité proximale 13.

Le logement 10 est associé à un dispositif de maintien 20 du système intra-utérin dans l'utérus. Ce dispositif de maintien 20 est agencé à l'extrémité proximale 13 du logement 10.

Dans ce mode de réalisation, le dispositif de maintien 20 comprend un tube cylindrique 21, un fil coudé formant ressort 22 et un bras de retenue 23.

Le tube cylindrique 21 est fixé à une de ces extrémités 21a à l'extrémité proximale 13 du logement 10.

En pratique, le bouchon proximal 15 comporte un prolongement 16 dont le diamètre est adapté à entrer à l'intérieur du tube cylindrique 21, au niveau d'une extrémité 21a.

Ce prolongement 16 est collé par exemple dans le tube cylindrique 21.

Le bouchon 15 est assemblé au logement 10 au niveau de l'extrémité proximale 13 par simple ajustement du logement 10 sur le bouchon 15.

En pratique, ce bouchon 15 peut présenter une forme tronconique permettant un ajustement étanche du bouchon 15 à l'extrémité 13 du logement 10.

Le tube cylindrique 21 est réalisé en un matériau biocompatible, comme le logement 10, et par exemple en silicone.

En pratique, ce tube cylindrique 21 peut avoir une longueur du même ordre que celle du logement 10, sensiblement égale à 10 mm et présenter un diamètre légèrement supérieur à celui du logement 10.

A titre d'exemples non limitatifs, le diamètre intérieur du tube cylindrique 21 peut être compris entre 0,5 et 0,6 mm et le diamètre extérieur du tube cylindrique 21 peut être compris entre 0,9 et 1 mm.

Le tube cylindrique 21 s'étend ainsi dans le prolongement du logement 10 suivant la direction de l'axe X.

Le fil coudé formant ressort 22 est monté dans le tube cylindrique 20 au niveau de sa seconde extrémité 21b.

En pratique, le fil coudé formant ressort 22 est solidarisé à l'intérieur du tube cylindrique 21.

En pratique, dans ce mode de réalisation, le fil coudé formant ressort 22 comporte une première extrémité 22a agencée dans le tube cylindrique 21, au niveau de l'extrémité proximale 13 du logement 10 et une seconde extrémité 22b.

La première extrémité 22a du fil coudé formant ressort 22 s'étend dans la direction de l'axe X dans le prolongement du logement 10 et du tube cylindrique 20.

La portion terminale 22'a de la première extrémité 22a est conformée en ressort hélicoïdal. Cette portion en forme de ressort hélicoïdal est solidarisée à l'intérieur du tube cylindrique 20, par exemple au moyen d'une colle en silicone.

La seconde extrémité 22b du fil coudé formant ressort 22 comprend une portion terminale recouverte d'un manchon de protection 24. Cette portion terminale de la seconde extrémité 22b recouverte du manchon de protection 24 constitue le bras de retenue 23 du dispositif de maintien, ce bras de retenue étant adapté à maintenir le dispositif dans la cavité utérine comme cela sera décrit ultérieurement.

Dans ce mode de réalisation particulier, le fil coudé formant ressort 22 comporte deux coudes 22c, 22d de telle sorte qu'une portion intermédiaire 22e s'étend entre la première extrémité 22a et la seconde extrémité 22b du fil coudé formant ressort 22.

En pratique, ce fil coudé formant ressort 22 peut être réalisé à partir d'un ressort en acier inox dont une première partie, correspondant à la portion terminale 22'a de la première extrémité 22a, est conformée en ressort hélicoïdal et une seconde partie est redressée et pliée pour réaliser une partie rectiligne de la première extrémité 22a, la partie intermédiaire 22a et la seconde extrémité 22b du fil coudé.

Ainsi, le bras de retenue 23 réalisé au niveau de la seconde extrémité 22b du fil coudé formant ressort 22 est agencé sur des moyens élastiques constitués principalement de la partie intermédiaire 22e et des coudes 22c et 22d du fil coudé formant ressort 22.

Comme bien illustré à la figure 1, ces moyens élastiques ont une force de rappel du bras de retenue 23 dans une position stable dans laquelle le bras de retenue 23 est écarté de l'axe X dans lequel s'étendent le logement 10, le tube cylindrique 20 et la première extrémité 22a du fil coudé formant ressort.

A titre d'exemples non limitatifs, le diamètre du fil d'acier utilisé pour former le fil coudé formant ressort peut être compris entre 0,1 et 0,2 mm.

La longueur de la portion 22'a en forme de ressort hélicoïdal peut être sensiblement égale à la moitié de la longueur du tube cylindrique 20, et par exemple égale à 5 mm.

Par ailleurs, le manchon de protection 24 peut être réalisé à partir d'un tube de nylon monté sur la portion terminale 22'b de la seconde extrémité 22b du fil coudé 22. Ce tube de nylon peut être collé, par exemple à l'aide d'une colle silicone, sur le fil d'acier.

A titre d'exemples non limitatifs, la longueur du manchon de protection peut être sensiblement égale à 5 mm, le diamètre extérieur étant compris entre 0,4 et 0,5 et le diamètre intérieur étant compris entre 0,15 et 0,25 mm pour permettre l'introduction du fil d'acier.

Outre ce dispositif de maintien 20 fixé au logement 10, le système intra-utérin récupérable comporte également un fil de retrait 30 permettant de retirer le dispositif de l'utérus par traction.

Ce fil de retrait 30 peut être en nylon et présenter une longueur totale de 150 mm et un diamètre de 0,1 mm.

Dans ce mode de réalisation, ce fil de nylon 30 est solidarisé à la portion terminale 22'a en forme de ressort hélicoïdal.

En pratique, le fil de nylon 20 peut être fixé à l'intérieur de la spirale du ressort hélicoïdal, avant l'introduction de la spirale dans le tube cylindrique 21. En chauffant cette portion terminale 22'a, on obtient la soudure du fil de nylon à l'intérieur de la spirale et sa solidarisation ainsi au fil coudé 22.

La portion terminale 22'a associée au fil de retrait 30 est ensuite introduite à l'intérieur du tube cylindrique 21 et collée comme indiqué précédemment au moyen d'une colle de silicone.

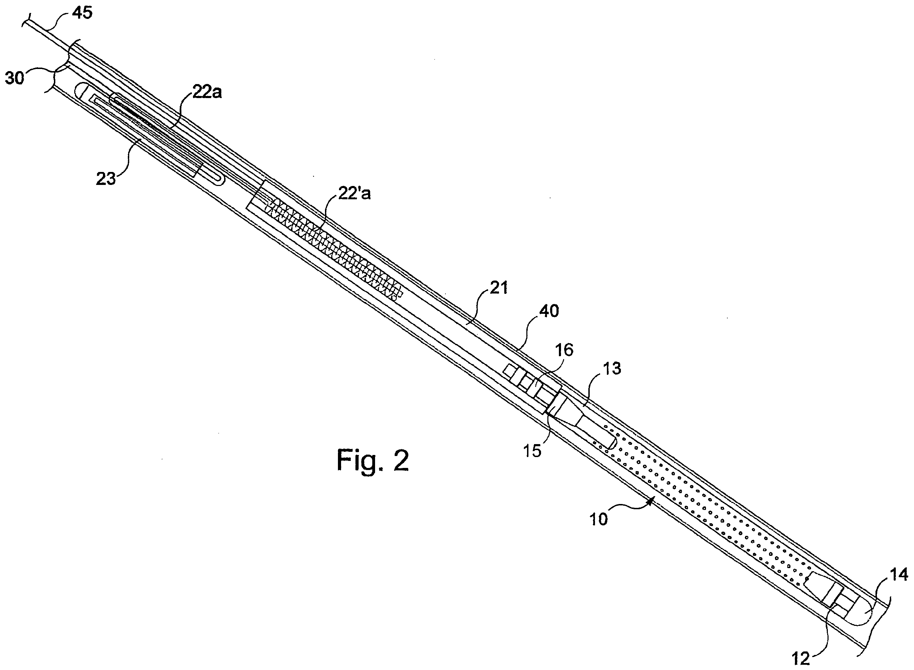

On va décrire à présent en référence aux figures 2 à 5, les différentes positions que peut prendre le bras de retenue 23 lors de la mise en oeuvre du système intra-utérin récupérable.

En pratique, le logement 10 est assemblé au dispositif de maintien, et plus particulièrement au tube cylindrique 21 après l'introduction des éléments dans le logement, et par exemple des gamètes mâles et femelles.

Lors de l'introduction de ces éléments, le bouchon distal 14 est en place au niveau de l'extrémité distale 12 du logement 10.

Après introduction des gamètes, le bouchon 15 est monté à l'extrémité 13 du logement 10, simultanément au montage du dispositif de maintien 20 du fait de la fixation du tube cylindrique 21 au niveau du prolongement 16 du bouchon proximal 15.

Pour permettre l'introduction du système intra-utérin dans la cavité utérine, on utilise, comme illustré à la figure 2, de manière traditionnelle, un cathéter 40 qui se présente globalement sous la forme d'un tube cylindrique allongé de longueur appropriée permettant de loger l'intégralité du système intra-utérin, c'est-à-dire successivement le logement 10, le tube cylindrique 20 et le fil formant ressort 22 associé au bras de retenue 23.

Par ailleurs, le fil de retrait 30 se prolonge au-delà du cathéter de transfert.

En outre, une tige semi rigide 45 est également utilisée en venant en appui sur le dispositif de maintien, au niveau de l'extrémité libre 21b du tube cylindrique 21.

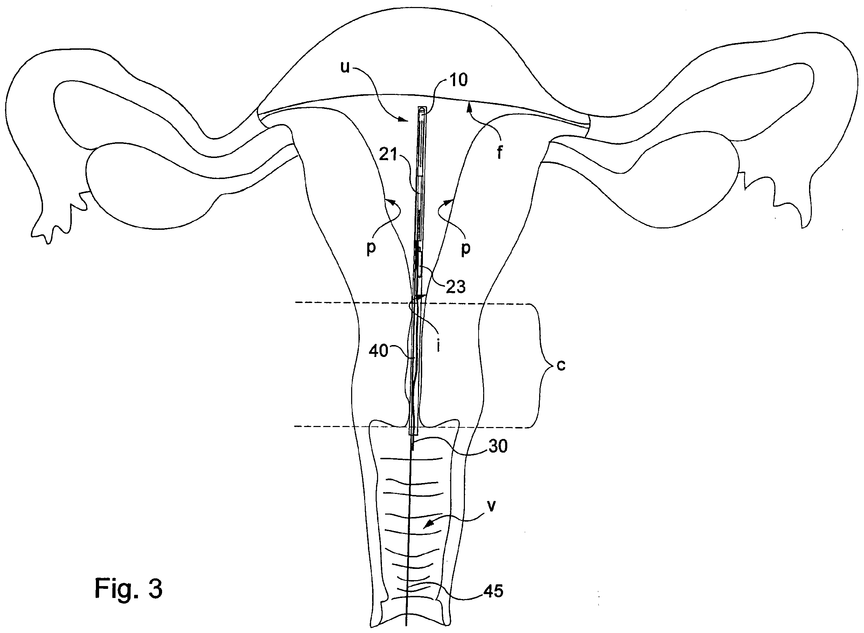

Cette tige semi rigide comme bien illustré à la figure 3 est suffisamment longue pour dépasser du cathéter au niveau de l'extrémité libre.

Dans cette position d'introduction, le bras de retenue 23, comme bien illustré à la figure 2, est sensiblement parallèle à l'axe X du logement 10 et disposé dans le prolongement de ce logement 10.

En pratique, dans cette position d'introduction, le fil coudé formant ressort est comprimé de manière à fermer les angles d'ouvertures au niveau des coudes 22c et 22d contre la force de rappel exercée par le fil formant ressort 22.

Dans cette position, les première et seconde extrémités 22a, 22b et la partie intermédiaire 22e sont adjacentes les unes aux autres et dans une direction parallèle à l'axe X.

Le bras de retenue 23 s'étend ainsi dans le prolongement du tube cylindrique 21 et du logement 10, au-delà de l'extrémité libre 21b du tube cylindrique 21, de manière à ne pas former de surépaisseur et permettre l'introduction de l'ensemble du système dans le cathéter de transfert 40.

Comme bien illustré à la figure 3, le système dans sa position d'introduction est introduit dans la cavité utérine u jusqu'à ce que l'ensemble du système s'étendent au-delà du col de l'utérus.

Plus précisément, l'ensemble du système dans le cathéter de transfert 40 traverse successivement le vagin v, puis le col c de l'utérus avant de déboucher dans la cavité utérine.

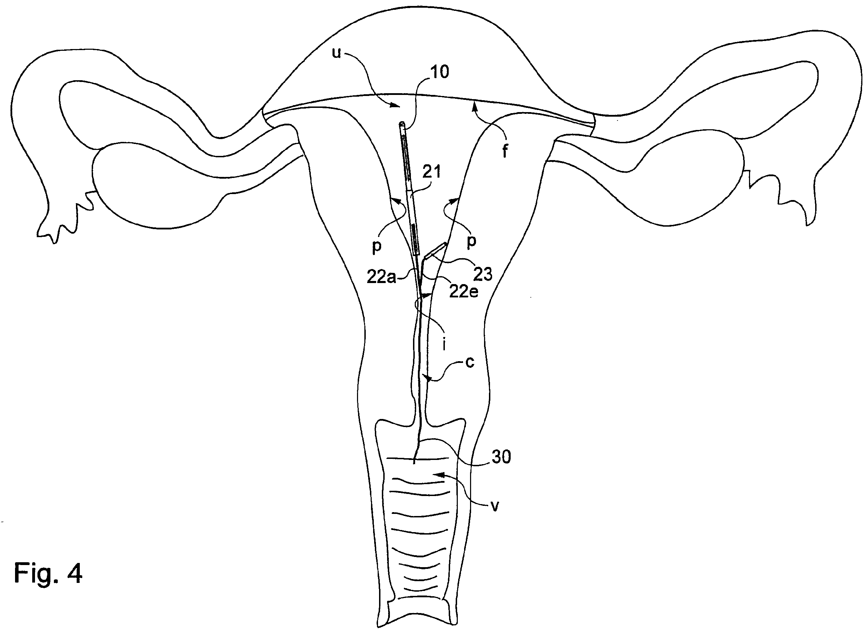

Après retrait du cathéter, comme illustré à la figure 4, les moyens élastiques du fil formant ressort 22 sont adaptés à positionner le bras de retenue 23 dans une position écartée de l'axe X du logement, correspondant à une position libre de maintien dans la cavité utérine.

Pour retirer le cathéter, le praticien maintient en position le logement 10 associé au dispositif de maintien 20 grâce à la tige semi rigide 45.

Tout en maintenant en place le dispositif grâce à la tige semi rigide, le praticien retire le cathéter 40 par translation le long de la tige semi rigide 45.

Une fois le cathéter entièrement retiré, la tige semi rigide 45 est également retirée, le logement 10 étant maintenu à l'intérieur de la cavité utérine notamment grâce au positionnement du bras de retenu 23 dans une position écartée de l'axe X du logement 10.

Comme bien illustré à la figure 4, le contact du bras de retenue 23 dans la cavité utérine est limité à la partie proche du col c de l'utérus, au niveau du canal isthmique i.

Enfin, lors du retrait du dispositif après une période de développement préimplantatoire, tel qu'illustré à la figure 3, le bras de retenue 23 est adapté à prendre une position de retrait dans laquelle il est sensiblement parallèle à l'axe X du logement.

En pratique, dans cette position de retrait, le bras de retenue 23 pivote autour d'un coude 22d reliant la seconde extrémité 22b à la partie intermédiaire 22e du fil coudé formant ressort 22.

Cette position de retrait du bras de retenue 23 sensiblement parallèlement à l'axe X est ainsi opposée à la position d'introduction.

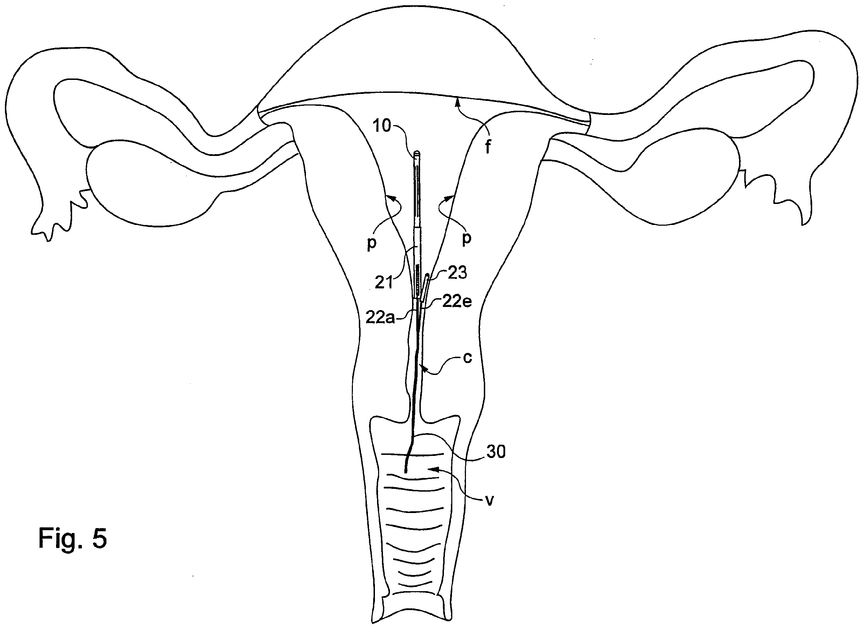

Comme bien illustré à la figure 5, lors du retrait du dispositif, le bras de retenue 23 ne vient en contact qu'avec une très faible portion de la paroi endométriale, au niveau du canal isthmique i.

Ainsi grâce au montage du bras de retenue 23 sur le fil coudé formant ressort 22, ce bras de retenue 23 peut prendre deux positions distinctes et opposées d'introduction et de retrait du système, permettant de limiter au mieux le contact entre ce bras de retenue et la paroi endométriale lors des déplacements du système.

Ainsi, la couche endométriale qui recouvre la cavité utérine au niveau de sa partie fundique f, la partie corporiale p et la partie du canal isthmique i, entre très peu en contact avec le dispositif introduit dans la cavité utérine u, et notamment le dispositif de maintien.

Celui-ci vient en contact principalement avec le col c de l'utérus, qui ne comporte pas de couche endométriale, et, dans une moindre mesure, avec le canal isthmique i.

Cette caractéristique est particulièrement importante dès lors qu'au moment de l'implantation de l'embryon, ce dernier est mis en contact avec la couche endométriale de l'utérus pour permettre la nidation et le développement de l'embryon dans l'utérus.

Bien entendu, la présente invention n'est pas limitée à l'exemple de réalisation décrit précédemment.

En particulier, le dispositif de maintien pourrait comporter plusieurs bras de retenue disposés par exemple symétriquement par rapport à l'axe longitudinal X du logement 10.

En outre, le type de montage du bras de retenue au niveau de l'extrémité proximale 13 du logement 10 n'est nullement limitatif.

Ainsi, le tube cylindrique 21 pourrait éventuellement être supprimé, le fil formant ressort 22 étant directement fixé au niveau de son extrémité 22a à l'extrémité proximale 13 du logement 10.

Par ailleurs, le fil formant ressort 22 ne pourrait ne comporter qu'un seul coude 22c, sans partie intermédiaire 22e, le coude 22c reliant directement la première partie 22a à la seconde partie 22b.

Dans un tel cas, le bras de retenue 23 serait adapté à n'occuper que deux positions, une position libre de maintien dans lequel le bras de retenue est écarté de l'axe X du logement 10 et une position d'introduction et de retrait dans lequel le bras de retenue est disposé sensiblement parallèlement à l'axe X du logement 10, dans le prolongement de ce logement 10 et du tube cylindrique 21.

## Revendications

1. Système intra-utérin récupérable comprenant un logement (10) adapté à contenir un ou plusieurs éléments choisis parmi le groupe comprenant un embryon, des gamètes mâles et/ou femelles, un ovocyte fécondé, un ovule non fécondé et une combinaison de ces éléments, et un dispositif de maintien (20) du système intra-utérin récupérable dans l'utérus, le logement (10) présentant, suivant son sens d'introduction selon un axe (X) dans une cavité utérine, une extrémité distale (12) et une extrémité proximale (13), **caractérisé en ce que** ledit dispositif de maintien (20) comprend au moins un fil coudé formant ressort (22) ayant une première extrémité (22a) agencée à ladite extrémité proximale (13) du logement (10) et une seconde extrémité (22b) solidaire d'un bras de retenue (23) dans la cavité utérine, ledit bras de retenue (23) étant adapté à prendre au moins deux positions :
- une position libre de maintien du logement dans une cavité utérine, dans laquelle ledit bras de retenue (23) est écarté dudit axe (X) ; et
- une position de retrait du logement de la cavité utérine, dans laquelle ledit bras de retenue (23) est sensiblement parallèle audit axe (X).

2. Système intra-utérin récupérable conforme à la revendication 1, **caractérisé en ce que** ledit bras de retenue (23) est adapté à prendre en outre au moins une position d'introduction dans laquelle ledit bras de retenue (23) est sensiblement parallèle audit axe (X) du logement (10) et dans le prolongement dudit logement (10).

3. Système intra-utérin récupérable conforme à l'une des revendications 1 ou 2, **caractérisé en ce que** ledit bras de retenue (23) est agencé sur des moyens élastiques (22) ayant une force de rappel adaptée à maintenir ledit bras de retenue (23) dans la position libre de maintien.

4. Système intra-utérin récupérable conforme à l'une des revendications 1 à 3, **caractérisé en ce que** ledit bras de retenue (23) est constitué par une portion terminale (22'b) de ladite seconde extrémité (22b) du fil coudé formant ressort (22), ladite portion terminale (22'b) étant recouverte d'un manchon de protection (24).

5. Système intra-utérin récupérable conforme à l'une des revendications 1 à 4, **caractérisé en ce que** ledit fil coudé formant ressort (22) comporte deux coudes (22c, 22d), ledit bras de retenue (23) étant adapté à prendre une position de retrait opposée à une position d'introduction par pivotement autour d'un desdits coudes (22d) du fil coudé formant ressort (22).

6. Système intra-utérin récupérable conforme à l'une des revendications 1 à 5, **caractérisé en ce qu'**une portion terminale (22'a) de ladite première extrémité (22a) du fil coudé formant ressort (22) est conformée en ressort hélicoïdal et solidarisée dans un tube cylindrique (21) en matériau biocompatible fixé à ladite extrémité proximale (13) dudit logement (10).

7. Système intra-utérin récupérable conforme à la revendication 6, **caractérisé en ce qu'**il comporte en outre un fil de retrait (30) dudit système fixé par insertion dans ledit ressort hélicoïdal (22'a).

8. Ensemble cathéter de transfert et système intra-utérin selon l'une quelconque des revendications 1 à 7, dans lequel ledit système est logé dans ledit cathéter.

## Patentansprüche

1. Entnehmbares intrauterines System, umfassend eine Aufnahme (10), die geeignet ist, ein oder mehrere Elemente zu enthalten, die aus der Gruppe ausgewählt sind, die einen Embryo, männliche und/oder weibliche Keimzellen, einen befruchteten Ovozyten, ein nicht befruchtetes Ovulum und eine Kombination dieser Elemente umfasst, und eine Haltevorrichtung (20) des entnehmbaren intrauterinen Systems im Uterus, wobei die Aufnahme (10) in ihrer Einführrichtung gemäß einer Achse (X) in einen Uterushohlraum ein distales Ende (12) und ein proximales Ende (13) aufweist, **dadurch gekennzeichnet, dass** die Haltevorrichtung (20) mindestens einen federbildenden gebogenen Draht (22) mit einem ersten Ende (22a), das an dem proximalen Ende (13) der Aufnahme (10) eingerichtet ist, und einem zweiten Ende (22b), das mit einem Haltearm (23) in dem Uterushohlraum fest verbunden ist, umfasst, wobei der Haltearm (23) geeignet ist, mindestens zwei Stellungen einzunehmen:
- eine freie Haltestellung der Aufnahme in einem Uterushohlraum, in welcher der Haltearm (23) von der Achse (X) beabstandet ist; und
- eine zurückgezogene Stellung der Aufnahme aus dem Uterushohlraum, in welcher der Haltearm (23) im Wesentlichen parallel zu der Achse (X) ist.

2. Entnehmbares intrauterines System nach Anspruch 1, **dadurch gekennzeichnet, dass** der Haltearm (23) geeignet ist, ferner mindestens eine eingeführte Stellung einzunehmen, in welcher der Haltearm (23) im Wesentlichen parallel zu der Achse (X) der Aufnahme (10) und in der Verlängerung der Aufnahme (10) ist.

3. Entnehmbares intrauterines System nach einem der Ansprüche 1 oder 2, **dadurch gekennzeichnet, dass** der Haltearm (23) auf elastischen Mitteln (22) mit einer Rückstellkraft eingerichtet ist, die geeignet ist, den Haltearm (23) in der freien Haltestellung zu halten.

4. Entnehmbares intrauterines System nach einem der Ansprüche 1 bis 3, **dadurch gekennzeichnet, dass** der Haltearm (23) aus einem terminalen Abschnitt (22'b) des zweiten Endes (22b) des federbildenden gebogenen Drahtes (22) besteht, wobei der terminale Abschnitt (22'b) von einer Schutzhülle (24) bedeckt ist.

5. Entnehmbares intrauterines System nach einem der Ansprüche 1 bis 4, **dadurch gekennzeichnet, dass** der federbildende gebogene Draht (22) zwei Bögen (22c, 22d) aufweist, wobei der Haltearm (23) geeignet ist, entgegen einer eingeführten Stellung durch Drehen um einen der Bögen (22d) des federbildenden gebogenen Drahtes (22) eine zurückgezogene Stellung einzunehmen.

6. Entnehmbares intrauterines System nach einem der Ansprüche 1 bis 5, **dadurch gekennzeichnet, dass** ein terminaler Abschnitt (22'a) des ersten Endes (22a) des federbildenden gebogenen Drahtes (22) als Spiralfeder ausgebildet ist und in einem zylindrischen Rohr (21) aus biologisch kompatiblem Material am proximalen Ende (13) der Aufnahme (10) befestigt ist.

7. Entnehmbares intrauterines System nach Anspruch 6, **dadurch gekennzeichnet, dass** es ferner einen Rückzugsdraht (30) des Systems aufweist, der durch Einsetzen in die Spiralfeder (22'a) befestigt ist.

8. Einheit Transferkatheter und intrauterines System nach einen der Ansprüche 1 bis 7, wobei das System in dem Katheter untergebracht ist.

## Claims

1. Recoverable intra-uterine system comprising a housing (10) suitable for containing one or more elements selected from the group comprising an embryo, male and/or female gametes, a fertilized oocyte, an unfertilized ovum and a combination of these elements, and a device for holding (20) the recoverable intra-uterine system in the uterus, the housing (10) having, along an axis (X) in the direction of introduction thereof into a uterine cavity, a distal end (12) and a proximal end (13), **characterized in that** said holding device (20) comprises at least one bent wire forming a spring (22) having a first end (22a) arranged at said proximal end (13) of the housing (10) and a second end (22b) firmly fixed to a retaining arm (23) in the uterine cavity, said retaining arm (23) being suitable for adopting at least two positions:
- a free position holding the housing in a uterine cavity, in which said retaining arm (23) is away from said axis (X); and
- a position for retracting the housing from the uterine cavity, in which said retaining arm (23) is substantially parallel to said axis (X).

2. Recoverable intra-uterine system according to claim 1, **characterized in that** said retaining arm (23) is also suitable for adopting at least one introduction position in which said retaining arm (23) is substantially parallel to said axis (X) of the housing (10) and within the extension of said housing (10).

3. Recoverable intra-uterine system according to one of claims 1 or 2, **characterized in that** said retaining arm (23) is arranged on elastic means (22) having a return force suitable for holding said retaining arm (23) in the free holding position.

4. Recoverable intra-uterine system according to one of claims 1 to 3, **characterized in that** said retaining arm (23) is constituted by an end portion (22'b) of said second end (22b) of the bent wire forming a spring (22), said end portion (22'b) being covered by a protective sleeve (24).

5. Recoverable intra-uterine system according to one of claims 1 to 4, **characterized in that** said bent wire forming a spring (22) includes two bends (22c, 22d), said retaining arm (23) being suitable for adopting a retraction position opposite to an introduction position by pivoting about one of said bends (22d) of the bent wire forming a spring (22).

6. Recoverable intra-uterine system according to one of claims 1 to 5, **characterized in that** an end portion (22'a) of said first end (22a) of the bent wire forming a spring (22) is shaped as a helical spring and firmly fixed in a cylindrical tube (21) of a biocompatible material fastened to said proximal end (13) of said housing (10).

7. Recoverable intra-uterine system according to claim 6, **characterized in that** it also includes a retraction wire (30) of said system fastened by insertion in said helical spring (22'a).

8. Transfer catheter and intra-uterine system set according to any one of claims 1 to 7, in which said system in housed in said catheter.
